# EUROPEAN PATENT APPLICATION

(11) **EP 1 743 654 A1**
(43) Date of publication of application: **17.01.2007**
(21) Application number: 05015439.2
(22) Date of filing: 15.07.2005
(51) Int. Cl.: A61K 45/06, A61P 35/00, A61K 31/19

(54) **Use of inhibitors of histone deacetylases in combination with NSAID for the therapy of cancer and/or inflammatory diseases**

(71) Applicant: TopoTarget Germany AG, 60596 Frankfurt am Main (DE)
(72) Inventor: MInk, Sigrun, Dr., 76135 Karlsruhe (DE); Martin, Elke, Dr., 76187 Karlsruhe (DE); Hentsch, Bernd, Dr., 60322 Frankfurt/Main (DE)
(74) Representative: Kalhammer, Georg

(57) **Abstract**

The present invention relates to the medical use of compounds acting as inhibitors of enzymes having histone deacetylase activity in conditions where their combination with compounds known as NSAID's, Non Steroidal Anti Inflammatory Drugs, causes an enhanced beneficial therapeutic effect. These conditions comprise cancer, cancer predisposing conditions, inflammatory and metabolic diseases. Furthermore, the invention includes the manufacture of clinically used medicaments for the therapy of the diseases mentioned herein, administering the compounds separately in the form of two individual drugs or in an administrative form which contains both drugs in a single application unit.

## Description

The present invention relates to the medical use of compounds acting as inhibitors of enzymes having histone deacetylase activity in conditions where their combination with compounds known as NSAID's, Non Steroidal Anti Inflammatory Drugs, causes an enhanced beneficial therapeutic effect. These conditions comprise cancer, cancer predisposing conditions, inflammatory and metabolic diseases. Furthermore, the invention includes the manufacture of clinically used medicaments for the therapy of the diseases mentioned herein, administering the compounds separately in the form of two individual drugs or in an administrative form which contains both drugs in a single application unit.

### BACKGROUND OF THE INVENTION

### Chromatin Regulation and Diseases

Local remodeling of chromatin is a key step in the transcriptional activation of genes. Dynamic changes in the nucleosomal packaging of DNA must occur to allow transcriptional proteins to make contact with the DNA template. One of the most important mechanisms influencing chromatin remodeling and gene transcription are the posttranslational modifications of histones and other cellular proteins by acetylation and subsequent changes in chromatin structure (Davie, 1998, Curr Opin Genet Dev 8, 173-8; Kouzarides, 1999, Curr Opin Genet Dev 9, 40-8; Strahl and Allis, 2000, Nature 403, 41-4). In the case of histone hyperacetylation, changes in electrostatic attraction for DNA and steric hindrance introduced by the hydrophobic acetyl group leads to destabilisation of the interaction of histones with DNA. As a result, acetylation of histones disrupts nucleosomes and allows the DNA to become accessible to the transcriptional machinery. Removal of the acetyl groups allows the histones to bind more tightly to DNA and to adjacent nucleosomes, and thus, to maintain a transcriptionally repressed chromatin structure. Acetylation is mediated by a series of enzymes with histone acetyltransferase (HAT) activity. Conversely, acetyl groups are removed by specific histone deacetylase (HDAC) enzymes. Disruption of these mechanisms gives rise to transcriptional misregulation and may contribute to a variety of human diseases, including autoimmune, inflammatory or hyperproliferative disorders including tumorigenic transformation and tumor progression.

Additionally, other molecules such as transcription factors alter their activity and stability depending on their acetylation status. E.g. PML-RAR, the fusion protein associated with acute promyelocytic leukemia (APL) inhibits p53 through mediating deacetylation and degradation of p53, thus allowing APL blasts to evade p53 dependent cancer surveillance pathways. Expression of PML-RAR in hematopoietic precursor cells results in repression of p53 mediated transcriptional activation, and protection from p53-dependent apoptosis triggered by genotoxic stresses (X-rays, oxidative stress). However, the function of p53 is reinstalled in the presence of HDAC inhibitors implicating active recruitment of HDAC to p53 by PML-RAR as the mechanism underlying p53 inhibition (Insinga et al., February 2004, EMBO Journal, 1-11). Therefore, acetylation of proteins distinct from histones, such as acetylation of p53, plays a crucial role in the anti-tumor activity of HDAC inhibitors.

### Nuclear Receptors and Histone Deacetylases

Nuclear hormone receptors are ligand-dependent transcription factors that control development and homeostasis through both positive and negative control of gene expression. Defects in these regulatory processes underlie the causes of many diseases and play an important role in the development of cancer. Many nuclear receptors, including T3R, RAR and PPAR, can interact with corepressors, such as N-CoR and SMRT, in the absence of ligand and thereby inhibit transcription. Furthermore, N-CoR has also been reported to interact with antagonist-occupied progesterone and estrogen receptors. Most interestingly, N-CoR and SMRT have been shown to exist in large protein complexes, which also contain mSin3 proteins and histone deacetylases (Pazin and Kadonaga, 1997; Cell 89, 325-8). Thus, the ligand-induced switch of nuclear receptors from repression to activation reflects the exchange of corepressor and coactivator complexes with antagonistic enzymatic activities.

### Gene Regulation by Nuclear Receptors

Such corepressor complexes which contain HDAC activity, not only mediate repression by nuclear receptors, but also interact with additional transcription factors including Mad-1, BCL-6, and ETO. Many of these proteins play key roles in disorders of cell proliferation and differentiation (Pazin and Kadonaga, 1997, Cell 89, 325-8; Huynh and Bardwell, 1998, Oncogene 17, 2473-84; Wang, J. et al., 1998, Proc Natl Acad Sci U S A 95, 10860-5). T3R for example was originally identified on the basis of its homology with the viral oncogene v-erbA, which in contrast to the wild type receptor does not bind ligand and functions as a constitutive repressor of transcription. Furthermore, mutations in RARs have been associated with a number of human cancers, particularly acute promyelocytic leukemia (APL) and hepatocellular carcinoma. In APL patients RAR fusion proteins resulting from chromosomal translocations involve either the promyelocytic leukemia protein (PML) or the promyelocytic zinc finger protein (PLZF). Although both fusion proteins can interact with components of the corepressor complex, the addition of retinoic acid dismisses the corepressor complex from PML-RAR, whereas PLZF-RAR interacts constitutively. These findings provide an explanation why PML-RAR APL patients achieve complete remission following retinoic acid treatment whereas PLZF-RAR APL patients respond very poorly (Grignani et al., 1998, Nature 391, 815-8; Guidez et al., 1998, Blood 91, 2634-42; He et al., 1998, Nat Genet 18, 126-35; Lin et al., 1998, Nature 391, 811-4).

Recently, a PML-RAR patient who had experienced multiple relapses after treatment with retinoic acid has been treated with the HDAC inhibitor phenylbutyrate, resulting in complete remission of the leukemia (Warrell et al., 1998, J. Natl. Cancer Inst. 90, 1621-1625).

### The Protein Family of Histone Deacetylases

The recruitment of histone acetyltranferases (HATs) and histone deacetylases (HDACs) is considered as a key element in the dynamic regulation of many genes playing important roles in cellular proliferation and differentiation. Hyperacetylation of the N-terminal tails of histones H3 and H4 correlates with gene activation whereas deacetylation can mediate transcriptional repression. Consequently, many diseases have been linked to changes in gene expression caused by mutations affecting transcription factors. Aberrant repression by leukemia fusion proteins such as PML-RAR, PLZF-RAR, AML-ETO, and Stat5-RAR serves as a prototypical example in this regard. In all of these cases, chromosomal translocations convert transcriptional activators into repressors, which constitutively repress target genes important for hematopoietic differentiation via recruitment of HDACs. It is plausible that similar events could also contribute to pathogenesis in many other types of cancer. There is growing evidence that the same holds true also for autoimmune, inflammatory or hyperproliferative disorders.

Mammalian histone deacetylases can be divided into three subclasses (Gray and Ekström, 2001). HDACs 1, 2, 3, and 8 which are homologues of the yeast RPD3 protein constitute class I. HDACs 4, 5, 6, 7, 9, and 10 are related to the yeast Hda 1 protein and form class II. Recently, several mammalian homologues of the yeast Sir2 protein have been identified forming a third class of deacetylases which are NAD dependent. Furthermore, HDAC11 has been classified as a class I histone deacetylase with structural features of a class II HDAC. All of these HDACs appear to exist in the cell as subunits of a plethora of multiprotein complexes. In particular, class I and II HDACs have been shown to interact with transcriptional corepressors mSin3, N-CoR and SMRT which serve as bridging factors required for the recruitment of HDACs to transcription factors.

### Therapy with HDAC Inhibitors

Additional clinical investigations have recently been initiated to exploit the systemic clinical treatment of cancer patients with the principle of HDAC inhibition. By now, a clinical phase II trial with the closely related butyric acid derivative Pivanex (Titan Pharmaceuticals) as a monotherapy has been completed demonstrating activity in stage III/IV non-small cell lung cancer (Keer et al., 2002, ASCO, Abstract No. 1253). More HDAC inhibitors have been identified, with NVP-LAQ824 (Novartis) and SAHA (Aton Pharma Inc.) being members of the structural class of hydroxamic acids tested in phase II clinical trials (Marks et al., 2001, Nature Reviews Cancer 1, 194-202). Another class comprises cyclic tetrapeptides, such as depsipeptide (FR901228 - Fujisawa) used successfully in a phase II trial for the treatment of T-cell lymphomas (Piekarz et al., 2001, Blood 98, 2865-8). Furthermore, MS-27-275 (Mitsui Pharmaceuticals), a compound related to the class of benzamides, is now being tested in a phase I trial treating patients with hematological malignancies.

### The HDAC inhibitor Valproic Acid

Valproic acid (VPA; 2-propyl-pentanoic acid) has multiple biological activities which depend on different molecular mechanisms of action:
- VPA is an antiepileptic drug.
- VPA is teratogenic. When used as an antiepileptic drug during pregnancy, VPA can induce birth defects (neural tube closure defects and other malformations) in a few percent of born children. In mice, VPA is teratogenic in the majority of mouse embryos when properly dosed.
- VPA activates a nuclear hormone receptor (PPARδ). Several additional transcription factors are also derepressed but some factors are not significantly derepressed (glucocorticoid receptor, PPARα).
- VPA occasionally causes hepatotoxicity, which may depend on poorly metabolized esters with coenzyme A.
- VPA is an inhibitor of HDACs.
- VPA induces proteasomal degradation of HDAC-2

The use of VPA derivatives allowed to determine that the different activities are mediated by different molecular mechanisms of action. Teratogenicity and antiepileptic activity follow different modes of action because compounds could be isolated which are either preferentially teratogenic or preferentially antiepileptic (Nau et al., 1991, Pharmacol. Toxicol. 69, 310-321). Activation of PPARδ was found to be strictly correlated with teratogenicity (Lampen et al., 1999, Toxicol. Appl. Pharmacol. 160, 238-249) suggesting that, both, PPARδ activation and teratogenicity require the same molecular activity of VPA. Also, differentiation of F9 cells strictly correlated with PPARδ activation and teratogenicity as suggested by Lampen et al., 1999, and documented by the analysis of differentiation markers (Werling et al., 2001, Mol. Pharmacol. 59, 1269-1276). It was shown, that PPARδ activation is caused by the HDAC inhibitory activity of VPA and its derivatives (WO 02/07722 A2; WO 03/024442 A2). Furthermore, it was shown that the established HDAC inhibitor TSA activates PPARδ and induces the same type of F9 cell differentiation as VPA. From these results it can be concluded that not only activation of PPARδ but also induction of F9 cell differentiation and teratogenicity of VPA or VPA derivatives are caused by HDAC inhibition.

The activity of VPA in promoting the selective proteasomal degradation of HDAC-2 is distinct form its activity as an inhibitor of the enzymatic activity of HDACs, as neither of the well characterized HDAC inhibitors Trichostatin A and MS-27-275 affect HDAC-2 degradation (Krämer et al, 2003, 22(13), 3411-3420).

Antiepileptic and sedating activities follow different structure activity relationships and thus obviously depend on a primary VPA activity distinct from HDAC inhibition. The mechanism of hepatotoxicity is poorly understood and it is unknown whether it is associated with formation of the VPA-CoA ester. HDAC inhibition, however, appears not to require CoA ester formation.

### Valproic Acid as inhibitor of histone deacetylases

VPA has been developed as a drug used for the treatment of epilepsia. Accordingly, VPA is used systemically, orally, or intravenously, to allow the drug to pass the blood brain barrier to reach the epileptic target regions in the brain tissue in order to fulfill its anti-epileptic mission. Moreover, VPA has been shown to possess beneficial effects when used for the treatment of many different types of human cancers as a single agent or in combination with a whole variety of other anti-tumor therapies which are individually based on strikingly different modes of action by inhibiting specific sets of enzymes having HDAC activity and thereby inducing differentiation and/or apoptosis (WO 02/07722 A2, EP 1170008; WO 03/024442 A2, EP 1293205 A1). For the treatment or prevention of malignant diseases autoimmune diseases or other inflammatory or hyperproliferative disorders, VPA may also be administered systemically, orally, or intravenously. Furthermore, it was shown, that VPA permeates human skin effectively and therefore can be administered topically on skin exhibiting beneficial effects when used for the topical treatment or prevention of autoimmune, inflammatory or hyperproliferative human skin diseases, e.g., psoriasis and human skin cancer (EP application No. 03014278.0).

### Non-Steroidal Anti-Inflammatory Drugs (NSAIDs) and their use

Non-Steroidal anti-inflammatory drugs (NSAIDs) are inhibitors of the cyclooxygenase enzymes, the key enzymes in the metabolism of arachidonic acid to prostaglandins and thromboxanes. Recently it was discovered that the cyclooxygenase enzymes comprise two isoforms: COX-1 and COX-2. COX-1 is constitutively expressed in most tissues where it contributes to physiologic functions. Its inhibition by NSAIDs has been associated with the common toxicities of these agents, including gastric ulceration and bleeding. COX-2 is an inducible enzyme that is induced in response to cytokines and growth factors at sites of inflammation and in some tumors, including but not limited to colon adenomas, colon and colorectal cancer, breast, lung and prostate tumors.
COX inhibitors are being used for the treatment of a variety of diseases and conditions, including pain, inflammatory disorders such as rheumatoid arthritis, but also for the inhibition of intestinal polyp growth, e.g., in patients suffering from Familial Adenomatous Polyposis (FAP). Epidemiologic studies have shown that people who have taken NSAIDs for prolonged periods of time have a lower than expected rate of colorectal adenomas and carcinomas.

Among this widely used class of NSAID's are aspirin, methyl salicylate, diclofenac [Voltaren®, Nu-Diclo®, Cataflam®], meclofenamate [Meclomen®], mefanamic acid [Ponstel®], meloxicam [Mobic®], nabumetone [Relafen®], naproxen [Aleve®, Anaprox®, Naprosyn®, Naprelan®, Naxen®, Novo-Naprox®, Synflex®], oxaprozin [Daypro®], phenylbutazone [Cotylbutazone®, Alka Butazolidine®], piroxicam [Feldene®, Nu-Pirox®], sulindac [Clinoril®, Novo-Sundac®], tenoxicam [Mobiflex®, diflunisal [Dolobid®], tiaprofenic acid [Albert Tiafen®, Surgam®], tolmetin [Tolectin®], etodolac [Lodine®], fenoprofen [Nalfon®], floctafenine [Idarac®], flurbiprofen [Ansaid®, Froben®], ibuprofen [Advil®, Dolgesic®, Excedrin®, Genpril®, Haltran®, Ibifon®, Ibren®, Ibu®, Ibuprin®, Ibuprohm®, Medipren®, Midol®, Motrin®, Nuprin®, Pamprin®, Q-Profen®, Rufen®, Trendar®], indomethacin [Indocin®, Indocid®], ketoprofen [Orudis®, Oruvail®, Actron®, Rhodis®], nimesulid [Aulin®]. A number of NSAIDs (named coxibs) selectively inhibit Cox-2 enzymatic activity: this subclass comprises celecoxib [Celebrex®, Celebra®, Onsenal®] rofecoxib [Vioxx®, Vioxx Dolor®, Ceoxx®] valdecoxib [Bextra®, Valdyn®], parecoxib [Dynastat®, Rayzon®] lumiracoxib [Prexige®], etoricoxib [Arcoxia®], deracoxib, tilmacoxib, robenacoxib, firocoxib and cimicoxib.

However, in December 2004, for one of the most prescribed rheumatoid arthritis and osteoarthritis pain medication Celebrex® (Celecoxib; Pfizer Inc.), a safety committee that was monitoring one of two five year drug trials of Celebrex® found from preliminary data that the patients who were taking high dosages of the drug were undergoing a somewhat increased risks of heart problems or strokes. These findings have led to termination of the studies.

Previously, Merck&Co. also had halted sales of its COX-2 inhibitor drug Vioxx® after similar side effects were obtained in clinical trials.

As mentioned above, COX enzymes regulate the levels of prostaglandins which modulate a variety of important functions in the body. One type of prostaglandin, e.g., helps line the stomach with a protective fluid called gastric mucosa. When the production of this protective fluid is diminished, some people are at risk for developing stomach ulcers.

COX-2 converts arachidonic acid in the body into prostaglandins. In cancer cells, COX-2 levels also rise and trigger production of prostaglandins. The prostaglandins bind to tumor cells and help turn on genes involved in the generation of new blood vessels, and thus supporting the cells' rapid growth.

Also, the relationship between NSAIDs, prostaglandins (PGs), proliferation and apoptosis was explored, e.g., in colon adenocarcinoma cells which express COX and synthesize PGs. The PG producing HT-29 colon cancer cells, e.g., were growth inhibited by the COX inhibitors sulindac and piroxicam. Colorectal cancer is the second most frequent cancer in the Western world, often lethal when invasion and/or metastasis occur. In addition to hepatocyte growth factor (HGF), colon cancer invasion is now believed to be driven also by prostaglandins, generated by the cyclooxygenase-2 (Cox-2) enzyme.

Thus, for many cells a link has been established between synthesis of prostaglandins and control of cell growth, such as also displayed in Balb/c 3T3 cells, where the epidermal growth factor-dependent proliferation is inhibited by the COX inhibitor indomethacin.

Also Aspirin which has been used to control pain and inflammation for over a century, has been linked by epidemiological studies with a decreased incidence of colorectal cancer with its long-term use in the early 1980s. Near the same time the first reports showing regression of colorectal adenomas in response to the NSAID sulindac were reported. In subsequent years, the use of other NSAIDs, which inhibit cyclooxygenase (COX) enzymes, was linked to reduced cancer risk in multiple tissues including those of the breast, prostate, and lung.

Today, the overexpression of COX-2, and also the upstream and downstream enzymes of the prostaglandin synthesis pathway, has been demonstrated in multiple cancer types and some pre-neoplastic lesions. Direct interactions of prostaglandins with their receptors through autocrine or paracrine pathways to enhance cellular survival or stimulate angiogenesis have been proposed as the molecular mechanisms underlying the procarcinogenic functions of Cyclooxygenase enzymes (For review see: Cancer Lett. 2004 Nov 8;215(1):1-20. Cyclooxygenases in cancer: progress and perspective. Zha S, Yegnasubramanian V, Nelson WG, Isaacs WB, De Marzo AM.).

In this respect recently also preclinical studies suggested that cyclooxygenase COX-2 may be involved in the molecular pathogenesis of some types of lung cancer. Most of the available studies point to its involvement in non-small cell lung cancer. Survival of patients with non-small cell lung cancer expressing high levels of COX-2 is markedly reduced. Treatment of humans with the selective COX-2 inhibitor Celecoxib augments the antitumor effects of chemotherapy in patients with non-small cell lung cancer. COX-2 has been shown to regulate some aspects of tumor-associated angiogenesis (Clin Cancer Res. 2004 Jun 15;10(12 Pt 2):4266s-4269s. Cyclooxygenase as a target in lung cancer. Brown JR, DuBois RN.).

In addition, several studies have suggested that cyclooxygenase-2 (COX-2) expression is associated with parameters of aggressive breast cancer, including large tumor size, positive axillary lymph node metastases, and HER2-positive tumor status. Studies of mammary tumors in mice and rats have indicated that moderate to high COX-2 expression is related to the genesis of mammary tumors that are sensitive to treatment with nonspecific and specific COX-2 inhibitors (Semin Oncol. 2004 Apr;31(2 Suppl 7):22-9. The role of COX-2 inhibition in breast cancer treatment and prevention. Arun B, Goss P.).

COX-2 is also highly expressed in prostate cancer, particularly in the epithelial cells of high-grade prostatic intraepithelial neoplasia and cancer. It was demonstrated that treatment of human prostate cancer cell lines with a selective COX-2 inhibitor induces apoptosis both in vitro and in vivo. The in vivo results also indicate that the COX-2 inhibitor decreases tumor microvessel density and angiogenesis. COX-2 inhibitors can prevent the hypoxic upregulation of a potent angiogenic factor, vascular endothelial growth factor. These results indicate that COX-2 inhibitors may, therefore, serve as effective chemopreventive and therapeutic agents in cancer of the prostate (Urology. 2001 Aug;58(2 Suppl 1):127-31. The role of cyclooxygenase-2 in prostate cancer. Kirschenbaum A, Liu X, Yao S, Levine AC).

### Combination of HDAC inhibitors and NSAID

WO 03/039599 A1 generally discloses combinations of cyclooxygenase-2 inhibitors with histone deacetylase inhibitors and their use in treating pre-malignant colon lesions or a colon cancer or other malignancies.

The inventors of the present application surprisingly found that the combination of HDAC inhibitors with inhibitors of COX enzymes is particularly useful in the treatment of diseases which are characterized by upregulation of the histone deacetylase HDAC-2. It has further been found that this combination results in a non-expected synergistic inhibition of downstream biological events which are also regulated by COX enzymes, such as the secretion of prostaglandins.

The present invention therefore relates to the use of a histone deacetylase inhibitor in combination with a NSAID for the manufacture of a medicament for the treatment or prevention of a disease wherein the disease is defined by upregulation of the histone deacetylase HDAC-2 in tissue affected by the disease.

The invention further relates to a method for the treatment or prevention of a disease which is characterized by upregulation of HDAC-2, comprising administering to an individual in need thereof an effective amount of a histone deacetylase inhibitor and an effective amount of a NSAID.

Another aspect of the invention is the use of a histone deacetylase inhibitor in combination with a NSAID for the manufacture of a medicament for the treatment or prevention of a disease in which the induction of hyperacetylation of histones has a beneficial effect, characterized in that at least one tissue of the individual to be treated shows upregulation of the histone deacetylase HDAC-2. When the disease is a tumor, the tissue is usually tumor tissue.

Upregulation of HDAC-2 in tissue affected by the disease may be the result of or associated with different mutations. APC mutations which lead to a lack of APC function, or beta-catenin mutations which lead to a gain of function of beta-catenin, may cause an increase in HDAC-2 levels. Similarly, upregulation or enhanced function of c-myc may lead to an upregulation of HDAC-2. Generally, mutations and alterations of the Wnt pathway may lead to upregulation of HDAC-2. Accordingly, the tissue affected by the disease may harbor at least one mutation in the APC gene. Alternatively, the tissue affected by the disease may harbor at least one mutation in the beta-catenin gene which leads to a gain of function of beta-catenin or a stabilization or enhanced half-life of beta-catenin protein. In yet another embodiment, the tissue affected by the disease shows upregulation or enhanced function of c-myc. In yet another embodiment, the tissue affected by the disease shows mutations and/or alterations of the Wnt pathway that lead to HDAC-2 upregulation.

In a preferred embodiment, the tissue affected by the disease harbors at least one mutation in the APC gene and at least one mutation in the beta-catenin gene. In another embodiment, the tissue affected by the disease harbors at least one mutation in the APC gene and at least one mutation in the beta-catenin gene and upregulation or enhanced function of c-myc.

The term "tissue" as used herein denotes biological material from the body of an individual. The term "tissue" includes cells obtained from the individual's body. The phrase "affected by the disease" refers to a situation where the cells or the tissue are different from the corresponding cells or tissue of a healthy individual. For example, the cells or tissue may exhibit uncontrolled cell division or harbor gene mutations which are not present in the corresponding cells of a healthy individual, or show signs of inflammation. In an optional first step, the tissue material may be provided by obtaining a biopsy from an individual's body.

The upregulation of HDAC-2 in the tissue affected by the disease to be treated is at least 10%, more preferably at least 25%, most preferably at least 50% as compared to the HDAC-2 level (protein or mRNA) of the corresponding tissue of a healthy individual. The term "upregulation" refers to the amount of HDAC-2 protein and/or HDAC-2 mRNA. Preferably, both protein and mRNA are upregulated.

Whether the tissue affected by a disease exhibits upregulation of the histone deacetylase HDAC-2, or the extent of upregulation, may be determined by measuring the amount and/or expression of HDAC-2. For example, the amount of HDAC-2 protein may be determined by an immunoassay using antibodies directed against HDAC-2. Such immunoassays are known to those of skill in the art. Antibodies directed against HDAC-2 are disclosed in Krämer et al. (2003) EMBO J. 22, 3411-3420 and can be obtained from various sources such as Santa Cruz Biotechnology, Inc., Zymed, SigmaAldrich and other companies.

Western Blotting may be used which is generally known in the art. The cellular material or tissue may be homogenized and treated with denaturing and/or reducing agents to obtain the samples. The sample may be loaded on a polyacrylamide gel to separate the proteins followed by transfer to a membrane or directly be spotted on a solid phase. The antibody is then contacted with the sample. After one or more washing steps the bound antibody is detected using techniques which are known in the art. Gel electorphoresis of proteins and Western Blotting is described in Golemis, "Protein-Protein Interactions: A Laboratory Manual", CSH Press 2002, Cold Spring Harbor N.Y.

Immunohistochemistry may be used after fixation and permeabilisation of tissue material, e.g. slices of solid tumors. The antibody is then incubated with the sample, and following one or more washing steps the bound antibody is detected. The techniques are outlined in Harlow and Lane, "Antibodies, A Laboratory Manual" CSH Press 1988, Cold Spring Harbor N.Y..

In a preferred embodiment, the amount of HDAC-2 protein is determined by way of an ELISA. A variety of formats of the ELISA can be envisaged. In one format, the antibody is immobilized on a solid phase such as a microtiter plate, followed by blocking of unspecific binding sites and incubation with the sample. In another format, the sample is first contacted with the solid phase to immobilize the HDAC-2 protein contained in the sample. After blocking and optionally washing, the antibody is contacted with the immobilized sample. ELISA techniques are described in Harlow and Lane, "Antibodies, A Laboratory Manual" CSH Press 1988, Cold Spring Harbor N.Y..

Alternatively, the amount of HDAC-2 mRNA or cDNA may be determined by nucleic acid technology as known to one of ordinary skill. Preferably hybridization techniques and/or PCR techniques are employed. Northern blotting techniques may be used to determine the amount of HDAC-2 RNA in the sample. In a preferred embodiment, RT-PCR is used. The person skilled in the art is able to design suitable primers and/or probes to be used in these methods on the basis of the nucleotide sequence of HDAC-2. The nucleotide sequence and suitable methods for determining the amount and/or expression of HDAC-2 are described in WO 2004/027418 A2.

According to one aspect of the use or method of the invention, a diagnostic step may be performed in order to determine whether an individual has a disease which is characterized by upregulation of HDAC-2. This diagnostic step may comprise determining in a tissue sample the amount or expression of HDAC-2 nucleic acid or protein. If the tissue sample exhibits upregulation of HDAC-2 the individual from whom the tissue was obtained can be treated according to the use or method of the invention.

The combination treatment according to the invention may therefore be preceded by the step of determining in a tissue sample the amount of or the expression of HDAC-2 nucleic acid (e.g. mRNA) or protein. Optionally, the individual may be selected if the amount of or the expression of HDAC-2 nucleic acid or protein in said sample is significantly higher (at least 10, 25 or 50 %) than that in a reference sample from a healthy individual.

It may further be determined whether or not mutations in the APC gene or in the beta-catenin gene are present. Further, the presence or absence of upregulation or enhanced function of c-myc can be determined in accordance with methods known in the art.

Several beta-catenin mutations in human cancers are disclosed in Table 1 of Polakis (2000) Genes & Development 14:1837-1851 (see page 1840 therein). These mutations are incorporated herein by reference. In one embodiment of this invention, the disease to be treated is characterized by at least one of these beta-catenin mutations in the tissue affected by the disease.

Mutations in the APC gene have been broadly described in the literature and are frequently associated with the development of gastrointestinal cancers, including cancers of the stomach, duodenum, colon and rectum, rendering the APC gene as a gatekeeper of colonic cancerogenesis (Behrens and Lustig, Int. J. Dev. Biol. 48: 477-487, 2004; and citations therein). Furthermore, APC mutations have also been found in a variety of additional cancers (Beroud and Soussi, Nucleic Acids Res. 24(1):121-4, 1996), including pancreatic cancers (Flanders and Foulkes, Med. Genet. 33: 889-898, 1996), thyroid cancers (Kurihara et al; Thyroid 14(12):1020-9, 2004), lung cancer (Ohgaki et al.; Cancer Lett. 207(2):197-203,-2004), kidney cancer (Pecina-Slaus et al., Pathology 36(2):145-51, 2004), melanoma (Worm et al., Oncogene 23(30):5215-26, 2004; Reifenberger et al., Int J Cancer 100(5):549-56, 2002). Furthermore, APC mutations in FAP and Turcot syndrome patients have also been associated with the development of medulloblastoma, papillary thyroid carcinoma, hepatoblastoma, and desmoid tumors (Lynch et al., Dig. Dis. Sci. 46(11):2325-32, 2001), as well as brain tumors (Sunahara et al., Nippon Rinsho 58(7):1484-9, 2000; Hamilton et al., N. Engl. J. Med. 332(13):839-47, 1995). The disclosure of these documents and the mutations described therein are incorporated herein by reference.

Preferably, the disease to be treated or prevented is an inherited condition leading to cancer. The diseases may further be cancer or an inflammatory disorder. In a particularly preferred embodiment, the inherited condition leading to cancer is Familial Adenomatous Polyposis (FAP).

One aspect of the invention is the use of a histone deacetylase inhibitor in combination with a NSAID for the manufacture of a medicament for treating or preventing an inflammatory disorder.

The disease to be treated or prevented may be an estrogen receptor-dependent breast cancer, estrogen receptor-independent breast cancer, hormone receptor-dependent prostate cancer, hormone receptor-independent prostate cancer, brain cancer, renal cancer, colon cancer, colorectal cancer, pancreatic cancer, bladder cancer, esophageal cancer, stomach cancer, genitourinary cancer, gastrointestinal cancer, uterine cancer, ovarian cancer, astrocytomas, gliomas, skin cancer, squamous cell carcinoma, Keratoakantoma, Bowen disease, cutaneous T-Cell Lymphoma, melanoma, basal cell carcinoma, actinic keratosis; ichtiosis; acne, acne vulgaris, sarcomas, Kaposi's sarcoma, osteosarcoma, head and neck cancer, small cell lung carcinoma, non-small cell lung carcinoma, leukemias, lymphomas and/or other blood cell cancers.

In another aspect the disease is rheumatoid arthritis, thyroid resistance syndrome, diabetes, thalassemia, cirrhosis, protozoal infection, rheumatoid spondylitis, all forms of rheumatism, osteoarthritis, gouty arthritis, multiple sclerosis, insulin dependent diabetes mellitus, non-insulin dependent diabetes, asthma, rhinitis, uveitis, lupus erythematoidis, ulcerative colitis, Morbus Crohn, inflammatory bowel disease, chronic diarrhea, psoriasis, atopic dermatitis, bone disease, fibroproliferative disorders, atherosclerosis, aplastic anemia, DiGeorge syndrome, Graves' disease, epilepsia, status epilepticus, Alzheimer's disease, depression, schizophrenia, schizoaffective disorder, mania, stroke, mood-incongruent psychotic symptoms, bipolar disorder, affective disorders, meningitis, muscular dystrophy, multiple sclerosis, agitation, cardiac hypertrophy, heart failure, reperfusion injury and/or obesity.

The NSAID to be used in the combination treatment may be a cyclooxygenase inhibitor, preferably it is a cyclooxygenase-2 inhibitor. Preferred NSAIDs in accordance with this invention are salicylates, arylalkanoic acids, 2-arylpropionic acids, N-arylanthranilic acids, oxicams such as meloxicam and piroxicam, coxibs such as celecoxib, valdecoxib, lumiracoxib, etoricoxib, and rofecoxib, sulphonanilides, indomethacin, sulindac, aspirin, flurbiprofen, ibuprofen, naproxen drugs, and derivatives thereof.

As used herein, the term "histone deacetylase inhibitor" denotes a substance that is capable of inhibiting the histone deacetylase activity of an enzyme having histone deacetylase activity.

The inhibitory activity of a histone deacetylase inhibitor can be determined in an in vitro assay as known to those skilled in the art (WO03/001403). The IC₅₀ value can be taken as a measure for the inhibitory activity of a histone deacetylase inhibitor. A low IC₅₀ value indicates a high inhibitory activity; a high IC₅₀ value indicates a low inhibitory activity. The histone deacetylase inhibitors used in accordance with this invention preferably have an IC₅₀ value of less than 1 mM, more preferably of less than 500 µM with respect to at least one histone deacetylase.

According to a preferred embodiment, the histone deacetylase inhibitor is capable of inhibiting preferentially a subset of histone deacetylases or selected deacetylases. The term "inhibiting preferentially" as used herein refers to a situation where a first group of histone deacetylases are inhibited more strongly than a second group of histone deacetylases by a given histone deacetylase inhibitor. Usually, the histone deacetylase inhibitor inhibiting preferentially a first group of histone deacetylases has an IC₅₀ value of less than 800 µM, preferably of less than 500 µM with respect to the histone deacetylases of said first group. The IC₅₀ value with respect to histone deacetylases of the second group is usually greater than 800 µM, preferably greater than 1 mM.

In a preferred embodiment, the histone deacetylase inhibitor is capable of inhibiting preferentially class I histone deacetylases. According to this first embodiment, class I histone deacetylases are inhibited more strongly than class II histone deacetylases. In this first embodiment, the histone deacetylase inhibitor usually has IC₅₀ values of less than 800 µM, preferably of less than 500 µM with respect to the histone deacetylase enzymes HDAC 1, 2, 3 and 8. In addition, the histone deacetylase inhibitor usually has IC₅₀ values of greater than 800 µM, preferably of greater than 1 mM with respect to the class II enzymes HDAC 4, 5, 6, 7, 9 and 10. In a specific embodiment, the histone deacetylase inhibitor to be used inhibits HDAC-2 stronger than the other histone deacetylases. It is most preferred that the histone deacetylase used in accordance with this invention has an IC₅₀ value of less than 800 µM, preferably of less than 500 µM with respect to HDAC-2.

Preferably, the histone deacetylase inhibitor used in accordance with this invention is capable of downregulating HDAC-2 protein or mRNA levels in cells treated therewith. This can be determined as described in Krämer et al. (2003) EMBO J. 22, 3411-3420, the disclosure of which is incorporated herein by reference. The downregulation may be at least 10% or at least 25% or at least 50%.

The histone deacetylase inhibitor used in the combination treatment of this invention may be a compound of formula I 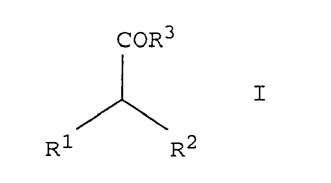 wherein R¹ and R² independently are a linear or branched, saturated or unsaturated, aliphatic C₃₋₂₅ hydrocarbon chain which optionally comprises one or several heteroatoms and which may be substituted, R³ is hydroxyl, halogen, alkoxy or an optionally alkylated amino group, or a pharmaceutically acceptable salt thereof. Preferably, R₁ and R₂ independently are a linear or branched C₃₋₂₅ hydrocarbon chain which optionally comprises one double or triple bond.

Most preferably, the histone deacetylase inhibitor is valproic acid or a pharmaceutically acceptable salt thereof.

In other aspects of the invention the histone deacetylase inhibitor may be selected from the group consisting of hydroxamic acid derivatives, benzamides, pyroxamides and derivatives thereof, microbial metabolites exhibiting HDAC inhibitory activity, fatty acids and derivatives thereof, cyclic tetrapeptides, peptidic compounds, HDAC class III inhibitors and SIRT inhibitors or a pharmaceutical acceptable salt thereof.

The hydroxamic acid derivative may be a compound such as NVP-LAQ824, LBH-589, MGCD0103, Trichostatin A (TSA), Suberoyl anilide hydroxamic acid, CBHA, G2M-701, G2M-702, G2M-707, Pyroxamide, Scriptaid, Cl-994, CG-1521, Chlamydocin, Biaryl hydroxamate, A-161906, Bicyclic aryl-N-hydroxycarboxamides, PXD-101, Sulfonamide hydroxamic acid, TPX-HA analogue (CHAP), Oxamflatin, Trapoxin, Depudecin, Apidicin, benzamides, MS-27-275, butyric acid and derivatives thereof, Pivanex (Pivaloyloxymethyl butyrate), trapoxin A, Depsipeptide (FK-228) and related peptidic compounds, Tacedinaline and MG2856 or a pharmaceutical acceptable salt thereof.

A preferred embodiment of this invention is the use of valproic acid in combination with Celecoxib (marketed e.g. as Celebrex®) (or other coxibs)) for the manufacture of a medicament for the prevention or treatment of a disease as defined herein above. Most preferably, valproic acid is used in combination with Celecoxib for treating FAP.

Another preferred embodiment of this invention is the use of valproic acid in combination with Sulindac (or other arylalkanoic acids) for the manufacture of a medicament for the prevention or treatment of a disease as defined herein above. Most preferably, valproic acid is used in combination with Sulindac for treating FAP.

Another preferred embodiment of this invention is the use of valproic acid in combination with aspirin (or other salicylates) for the manufacture of a medicament for the prevention or treatment of a disease as defined herein above. Most preferably, valproic acid is used in combination with aspirin for treating FAP.

Another preferred embodiment of this invention is the use of valproic acid in combination with lbuprofen (or other 2-arylpropionic acids) for the manufacture of a medicament for the prevention or treatment of a disease as defined herein above. Most preferably, valproic acid is used in combination with lbuprofen for treating FAP.

Another preferred embodiment of this invention is the use of valproic acid in combination with fenamic acid (or other N-arylanthranilic acids) for the manufacture of a medicament for the prevention or treatment of a disease as defined herein above. Most preferably, valproic acid is used in combination with fenamic acid for treating FAP.

Another preferred embodiment of this invention is the use of valproic acid in combination with piroxicam (or other oxicams) for the manufacture of a medicament for the prevention or treatment of a disease as defined herein above. Most preferably, valproic acid is used in combination with piroxicam for treating FAP.

In a specific aspect, the present invention relates to a combination, such as a combined preparation or a pharmaceutical composition, which comprises (a) valproic acid or a pharmaceutically acceptable salt thereof, and (b) Celecoxib. In another specific aspect, the present invention relates to a combination, such as a combined preparation or a pharmaceutical composition, which comprises (a) valproic acid or a pharmaceutically acceptable salt thereof, and (b) Sulindac. In another specific aspect, the present invention relates to a combination, such as a combined preparation or a pharmaceutical composition, which comprises (a) valproic acid or a pharmaceutically acceptable salt thereof, and (b) aspirin. In another specific aspect, the present invention relates to a combination, such as a combined preparation or a pharmaceutical composition, which comprises (a) valproic acid or a pharmaceutically acceptable salt thereof, and (b) lbuprofen. In another specific aspect, the present invention relates to a combination, such as a combined preparation or a pharmaceutical composition, which comprises (a) valproic acid or a pharmaceutically acceptable salt thereof, and (b) fenamic acid. In another specific aspect, the present invention relates to a combination, such as a combined preparation or a pharmaceutical composition, which comprises (a) valproic acid or a pharmaceutically acceptable salt thereof, and (b) piroxicam.

The active ingredients (a) and (b) may be present in free form or in the form of a pharmaceutically acceptable salt, for a simultaneous, concurrent, separate or sequential use. The parts of the kit of parts may be administered simultaneously or chronologically staggered, that is at different time points and with equal or different time intervals for any part of the kit of parts.

The medicament according to the invention may be applied by intravenous, intramuscular, subcutaneous, topical, oral, nasal, intraperitoneal or suppository-based administration.

The different drug compounds may be administered in the form of two individual drugs or in an administrative form which contains both drugs in a single application unit. The different drug compounds may be administered simultaneously or chronologically staggered, that is at different time points and with equal or different time intervals for any compound of the combination.

It has surprisingly been found that therapeutic doses of a NSAID and histone deacetylase inhibitor can be significantly reduced as compared to the respective monotherapies with these compounds.

Therefore, the preferred dosage of an NSAID when used in combination with an inhibitor of histone deacetylases may be reduced to 30-60% of the recommended or approved dose, more preferably to 60-80%, and more preferably to 80-90%.

In a specific aspect, the present invention relates to a combination, such as a combined preparation or a pharmaceutical composition, which comprises (a) valproic acid or a pharmaceutically acceptable salt thereof, and (b) Celecoxib, where the daily dose of celecoxib for the treatment is between 100 mg and 600 mg, and the daily dose of valproic acid or a pharmaceuticaliy acceptable salt thereof is between 10 mg/kg body weight and 60 mg/kg body weight. More preferable, the daily dose for celecoxib is between 200 mg and 500 mg, and the daily dose of valproic acid or a pharmaceutical acceptable salt thereof is between 20 mg/kg body weight and 45 mg/kg body weight. Most preferably, these combinations are used for treating FAP.

In another specific aspect, the present invention relates to a combination, such as a combined preparation or a pharmaceutical composition, which comprises (a) PXD101 or a pharmaceutically acceptable salt thereof, and (b) Celecoxib, where the daily dose of celecoxib for the treatment is between 100 and 600 mg, and the daily dose of PXD101 is between 300 mg and 10 g. More preferable, the daily dose for celecoxib is between 200 and 500 mg, and the daily dose of PXD101 is between 500 mg and 5 g. Most preferably, these combinations are used for treating FAP.

In another embodiment of the invention, the preferred dosage of an histone deacetylase inhibitor when used in combination with an NSAID may be reduced to 30-60% of the recommended or approved dose for the drug, more preferably to 60-80%, and more preferably to 80-90%.

In the light of the previously already known side effect profile of these drugs, in addition with recent data now also adding a potential increased risk of cardiotoxicity and stroke to this list of possible side effects, it may be required to lower the dose levels of these drugs, particularly if a long term or even chronical application is required. This, in fact, may be achieved by novel therapeutic ways of combining other therapeutic approaches with the use of COX inhibitors, thus allowing a decreased dosage of COX inhibitors drugs by maintaining at least the same therapeutic success with reduced side effects, or even enhancing the patients' benefit.

In this invention we present data showing that HDAC inhibitors can downregulate the expression of COX-2, and can thus, inhibit the cellular secretion of prostaglandins. This in turn contributes to the anti-cancerous properties of such HDAC inhibitors. However, and most importantly, now in this present invention we show that surprisingly the combination of HDAC inhibitors with inhibitors of COX enzymes results in a non-expected synergistic inhibition of downstream biological events which are also regulated by COX enzymes, such as the secretion of prostaglandins. These synergistic activities may be in part due to the enzymatic inhibition caused by the COX inhibitors, and secondly by the down regulation of the expression of the COX genes. However, since both mechanisms involve the same target structure it can be expected that this explanation is not sufficient to explain the observed synergistic effects, particularly seen in the most relevant in vivo test systems in animals. Thus, it must be concluded that additional surprising activities, most likely connected to the activity of inhibition of histone deacetylases are attributable to cause such beneficial synergistic effects. These findings can now be transferred to human clinical testing in order to provide patients suffering from cancer or inflammatory disorders a novel therapeutic option based on the combinational therapy using HDAC inhibitors together with COX inhibitors.

In addition, it can be expected that based on this combined synergistic activity it may be possible to lower the doses used for the NSAID's when used in combination with HDAC inhibitors which would result in a decrease of side effects related to the use of these NSAIDs.

Thus, as in the situation of Familial Adenomatous Polyposis (FAP), a combination of HDAC inhibitors and NSAIDs is expected to lead to a synergistic reduction of intestinal polyp growth and polyp burden. As a result, the currently standard of care for FAP patients, namely a prophylactic colectomy (surgical removal of the gut) may be postponed, potentially for years. In addition, since individual polyps in these patients finally progress to colon cancer, this progression may be suppressed and delayed.

Furthermore, this invention covers the use of a combination of HDAC inhibitors with COX inhibitors for the therapy of a whole variety of cancer indications, including but not limited to cancer of the colon, breast, lung and prostate.

Also, based on the anti-inflammatory activity of HDAC inhibitors those could be employed in combination with anti-inflammatory acting COX inhibitors to enable a novel therapeutic option that combines both inhibitory concepts to achieve additive or even synergistic therapeutic benefits in inflammatory disorders.

### EXAMPLES

### Example 1

Down regulation of the RNA and protein expression of COX-2 by inhibitors of histone deacetylases.

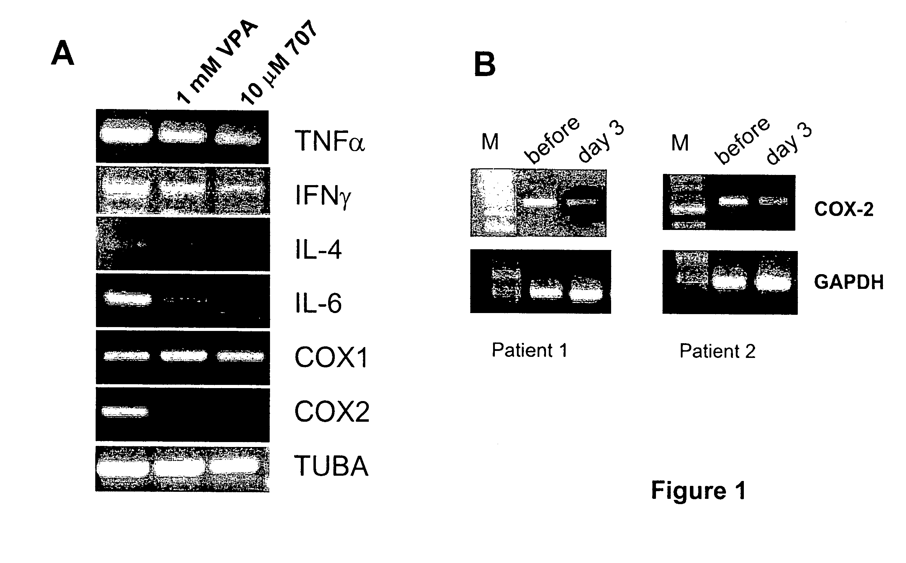

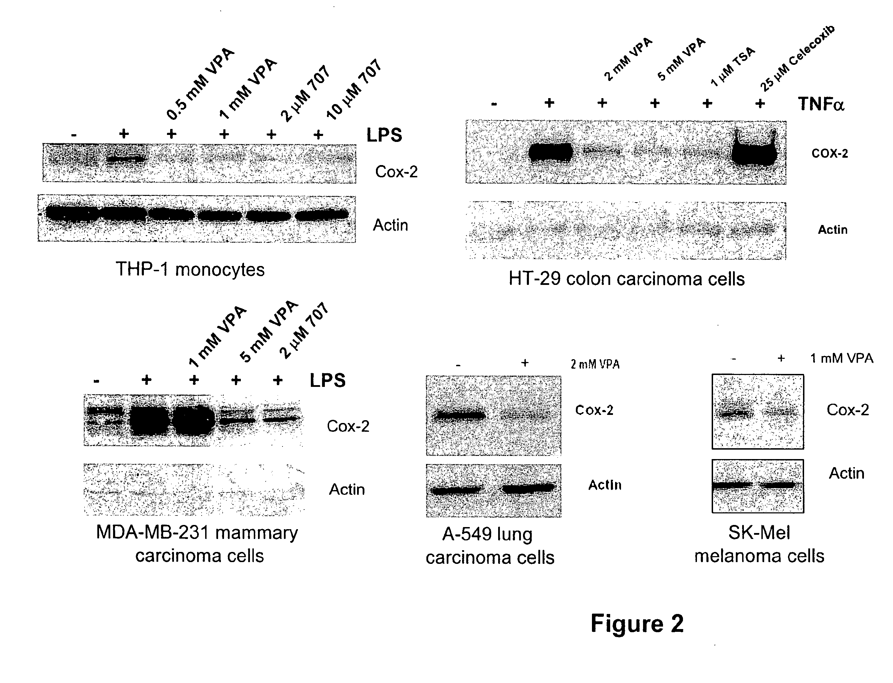

Expression of Cox-2 is downregulated by HDAC inhibitors (Figures 1 and 2). This could be shown for the HDAC inhibitory compounds valproic acid (VPA, TSA, G2M-701, G2M-702 and G2M-707 (see WO 2004/009536 A1 for details on G2M-701, G2M-702 and G2M-707) on RNA and protein level in several systems, such as A-549 human lung epithelial cancer cells, SK-Mel melanoma cells, HT-29 colon carcinoma cells, MDA-MB-231 mammary carcinoma cells, THP-1 monocytes and primary human lymphocytes and macrophages. Cox-1 levels analyzed at the same time are not affected as shown in figure 1. In contrast, the COX-2 inhibitor Celecoxib (Figure 2) does not alter the expression of COX-2.

THP-1 cells were induced to differentiate by addition of 20 ng/ml TPA to the growth medium for 3d. Adherent cells were seeded at a density of 5x10⁵ cells per well of a 6-well plate, and were incubated with 1 mM VPA or 10 µM G2M-707 over night (Figure 1A). Cox-2 expression was then induced by addition of 10 µg/ml LPS for 6h. RNA was prepared using the RNeasy Kit from Qiagen according to the manufacturer's instructions. Reverse transcription was done with 1 µg of RNA in a volume of 20 µl. 1 µl was used for each PCR reaction with specific primers for the indicated genes. Besides downregulation of TNF-α, IFN-γ and IL-6, also a downregulation of COX-2 RNA but not of COX-1 RNA by both HDAC inhibitors could be observed (Figure 1A).

Semi quantitative RT-PCR was also performed with RNA isolated form peripheral blood lymphocytes of patients treated with doses 30 mg/kg/d (Patient 1) or 120 mg/kg/d VPA (Patient 2) respectively. Figure 1B clearly shows a downregulation of Cox-2 but not of the control gene GAPDH by VPA treatment.

Regulation of Cox-2 on the protein level by HDAC inhibitors is shown in various cell types in figure 2. Here, A549 cells and SK-Mel melanoma cells showed constitutive expression of COX-2 and were not further induced. In HT-29 colon carcinoma cells Cox-2 expression was induced by treatment with 100 ng/ml TNF-α for 4h. For MDA-MB-231 mammary carcinoma cells and THP-1 monocytes 10 µg/ml LPS was used as an inductor for Cox-2 expression for 16h or 6h, respectively. HDAC inhibitor treatment was done for 72h (A-549), 48h (SK-Mel), starting 16h before induction (for THP-1 cells), or 30 min before induction (for HT29 and MDA-MB-231 cells).
Cells were seeded at densities between 5x10⁴ and 1x10⁵ cells per well of 24 well plates. Lysis was done by removing growth medium and adding 200 µl of Laemmli sample buffer per well. 60 µl were loaded on 8% acrylamide gels and subjected to discontinuous electrophoresis. Proteins blotted onto PVDF membranes were probed with a goat-anti-Cox-2 antibody (St.Cruz, sc1747) or a mouse anti-pan-actin antibody (Ab-5, NeoMarkers). In all systems a downregulation of Cox-2 protein but not of the control protein Actin by HDAC inhibitors could be observed (Figure 2).

### Example 2

Inhibition of prostaglandin secretion by inhibitors of histone deacetylases and their combination with inhibitors of COX enzymes (NSAIDs).

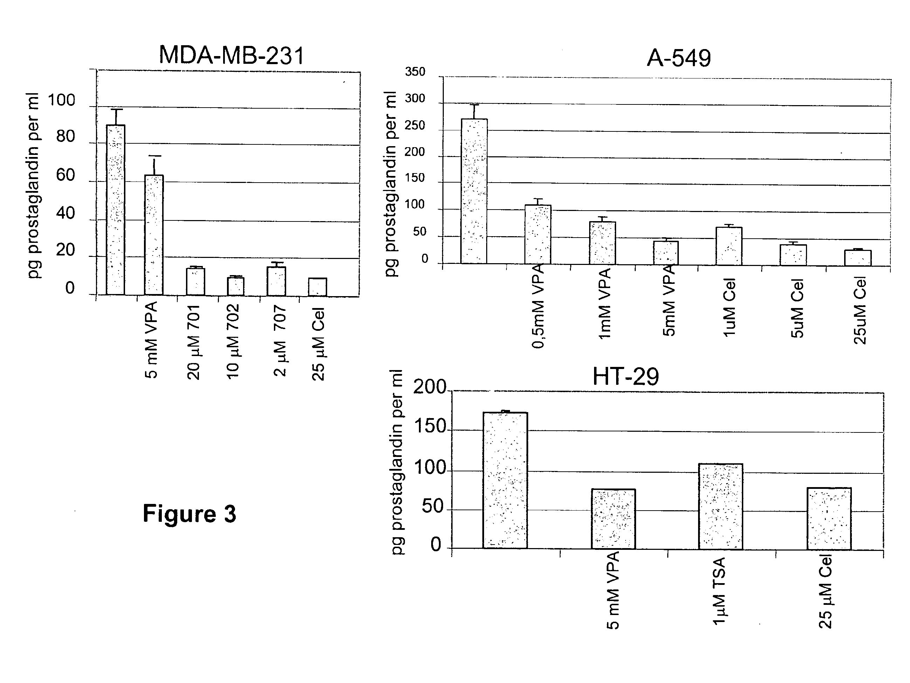

Inhibition of Cox-2 protein level by HDAC inhibitors results also in downregulation of secreted prostaglandin in several systems. This reduction of prostaglandin reaches the same level as with the Cox-2 inhibitor Celecoxib (Cel) as shown in figure 3.

In HT-29 colon carcinoma cells Cox-2 expression was induced by treatment with 100ng/ml TNF-α for 4h, for MDA-MB-231 mammary carcinoma cells 10 µg/ml LPS was used as an inductor for Cox-2 expression for 16h. HDAC inhibitor and Cox inhibitor treatment was done for 30 min before induction (HT-29, MDA-MB-231) or 16 h before lysis (A549). Prostaglandin levels in the supernatants were analyzed with the prostaglandin E2 EIA Kit from Cayman according to the manufacturer's instructions. Bars show the mean of two values, error bars reflect the range of the two values (Figure 3).

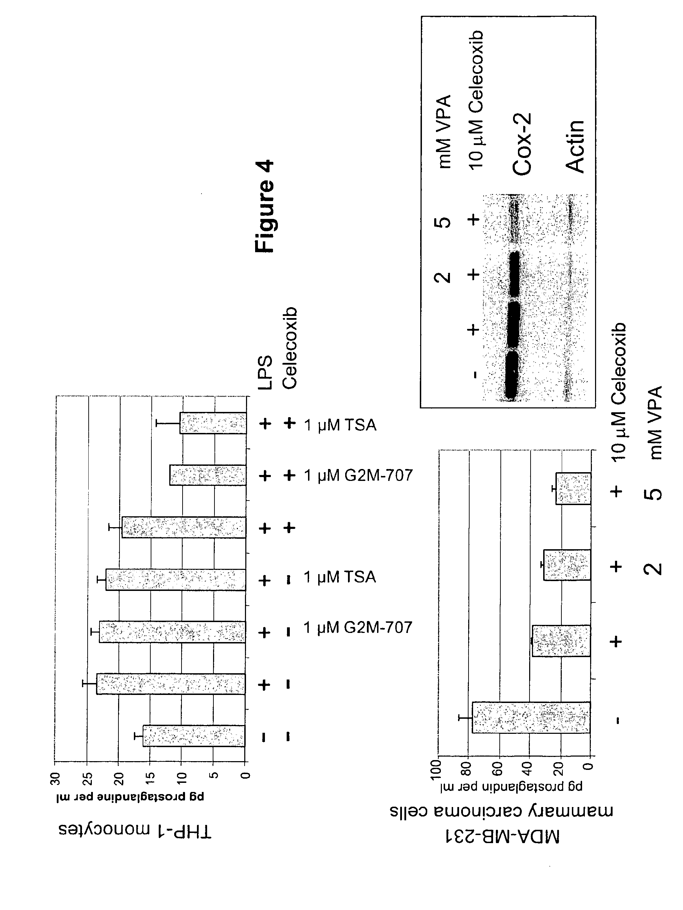

HDAC inhibitors could even enhance the reduction of prostaglandin secretion caused by the Cox inhibitor Celecoxib in THP-1 monocytes and MDA-MB-231 mammary carcinoma cells as shown in figure 4. In THP-1 monocytes the HDAC inhibitors G2M-707 and TSA could reduce the prostaglandin levels further, even after they already have been repressed by Celecoxib. This enhanced inhibition of prostaglandin secretion must be regarded as synergistic, since the use of combinations of Celecoxib and HDAC inhibitors results in a much more pronounced inhibition of prostaglandin secretion than an only on adding of the individual inhibitory activities would have suggested. Thus, the HDAC inhibitory function of these HDAC inhibitors appears to surprisingly support the COX-inhibitory function in down regulating the prostaglandin secretion by a so far undiscovered mechanism which allows these synergistic results. Also, in MDA-MB-231 cells the HDAC inhibitor VPA could dose dependently enhance the repression of prostaglandin secretion by Celecoxib. In parallel, Cox-2 protein levels were reduced as already described.

In detail, cells were seeded at a density of 7,5 x10⁴ per well in 24 well plates. Supernatants were analyzed in a dilution of 1:3 in duplicates with the prostaglandin E2 EIA Kit from Cayman according to the manufacture's instructions. Bars show the mean of two values. Cell extracts were prepared by removing growth medium completely and adding of 200 µl of Laemmli Sample buffer per well. Subsequently, 60 µl were loaded on 8% acrylamide gels and subjected to discontinuous electrophoresis. Proteins blotted onto PVDF membranes which were probed with a goat anti-Cox-2 antibody (St.Cruz, sc1747) or a mouse anti-pan-actin (to analyze the expression of this control protein) antibody (Ab-5, NeoMarkers).

### Example 3

Synergistic inhibition of adenoma growth in vivo by using inhibitors of histone deacetylases in combination with inhibitors of Cox-2.

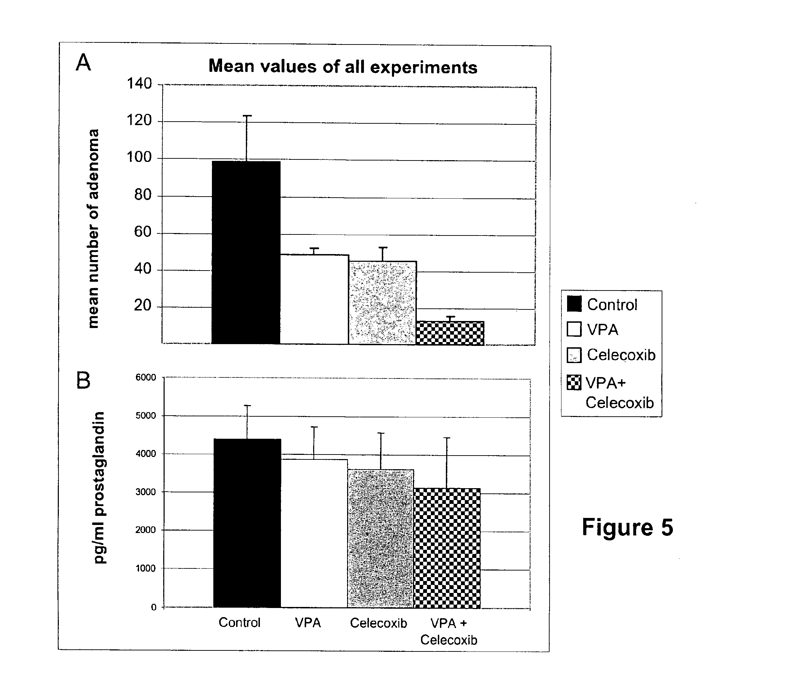

Treatment with VPA significantly reduces the number of adenomas in the APC^{min} mouse model. Similar results were obtained by utilizing the Cox-2 inhibitor Celecoxib in this model. However, upon combination therapy using both drugs in this model at the same time, a synergistic reduction in numbers of adenomas was observed. This, again, argues strongly, that the dual activity of VPA, its ability to down regulate Cox-2 protein levels, and its HDAC inhibitory function are responsible for the observed synergistic therapeutic effect when employed together with classical Cox-2 inhibitors (Figure 5A).

Shown are mean values of 15 (control group), 17 (VPA group), 13 (Celecoxib group) or 5 (combination treatment group) animals per group with standard error bars. P < 0,05 (two-sample t-test; Control vs. VPA- and Celecoxib-treated and monotherapy vs. combination therapy-treated animals.

Figure 5b shows prostaglandin levels in liver extracts of APC^{min} mice (an animal model of Familial Adenomatous Polyposis, an inherited disease which leads to the development of colon cancer) after treatment with VPA and Celecoxib alone and after treatment with the combination of both drugs. Here, it could be shown that both drugs decrease the levels of prostaglandins to a similar extent. Using both drugs in combination therapy at the same time resulted in an additive decrease of prostaglandin secretion. This enhanced suppression of prostaglandin secretion was additive, again arguing that the observed synergism in the reduction of adenoma growth can not solely be explained by interfering with the inhibition of prostaglandin secretion, but rather must be attributed to the dual activity of VPA, (i) acting as an inhibitor of HDAC enzymes, and (ii) its ability to down regulating the expression of Cox-2 with a subsequent reduction of prostaglandin levels.

Shown are mean values of two (control group), four (VPA group) and five (celecoxib and combination groups) animals with standard deviations.

In detail, seven to sixteen weeks old age- and sexmatched heterozygous C57BL/6J-APC^{min/+} mice (Jackson Laboratories, Bar Harbor, Maine) were either left untreated or were treated with VPA or Celecoxib or both drugs, respectively. Control animals were injected (i.p.) with PBS. VPA was injected (i.p.) as isotonic aqueous solution of its sodium salt (2x400 mg/kg/day) for four weeks, while Celecoxib was fed to the animals *ad libitum* with their diet at 1250 ppm (0,12%) for four weeks. The combination group received the same dosage as the single treatment groups of both, the VPA and the Celecoxib groups, for four weeks. At sacrifice entire intestinal tracts were opened longitudinally and fixed in 10% phosphate buffered formaldehyde for 24 hours followed by ethanol-fixation in 50% ethanol for three days. Polyp contrast was increased performing a 1 min staining in 0, 1 % methylene blue prior to determination of polyp numbers and sizes under a dissecting microscope by two independent observers unaware of the treatment that the mice had received.

Cyclooxygenase activity was assessed *ex vivo* in hepatic tissue as described (Reuter et al., 2002; BMC Cancer 2:19). Briefly, mice were euthanized 3 hours following the final dose of vehicle or drugs and a sample of liver tissue (~100 mg) was obtained. The samples were then placed into microcentrifuge tubes containing 1 ml of sodium phosphate buffer (10 mM, pH 7.4) and finely minced with scissors for 15 seconds. Samples were then incubated for 20min at 37° in a shaking water bath. After the incubation period, samples were centrifuged at 9000 x g for 30 seconds and the supernatants collected. Supernatants were flash frozen in liquid nitrogen and stored at -80°C for subsequent determination of prostaglandin E2 content. PGE2 concentrations were determined in duplicates after 1:20 dilution of supernatants using a commercially available competitive Enzyme Immunoassay (Cayman Chemical).

### Example 4

HDAC inhibitors reduce clinical severity scores in a therapeutic model of Collagen induced Rheumatoid Arthritis (CIA).

Cox-2 is known to be central to the inflammatory process (Dubois R. et al., FASEB J 12, 1063-1073 (1998)). It is rapidly upregulated by the inflammation mediator TNF-α and the prostaglandins produced by COX enzymes further suppress immunosurveillance. Thus inhibition of COX enzymes and subsequent decrease of prostaglandin production finally leads to relief of inflammatory symptoms and thus exploits its palliative effects. However, it does not effectively affect the cause of the inflammatory process. In recent years the search for a rather causal therapy of inflammatory diseases resulted in the design of novel therapies targeting TNF-α as the central inflammation mediator.
Recently, it was discovered that HDAC inhibitors display anti-inflammatory activity (see also Figure 1 in which HDAC inhibitors down regulate the expression of inflammatory cytokines, including TNF-α). Therefore, it can be proposed that HDAC inhibitors may be employed in combination with anti-inflammatory acting Cox-inhibitors to enable a novel therapeutic option that combines both inhibitory concepts to achieve additive or even synergistic therapeutic benefits when treating inflammatory disorders. Here, in particular and as mentioned above, the dual mechanism of HDAC inhibitors, namely their HDAC inhibitory activity and their ability to down regulate Cox-2 expression and thus, to subsequently decrease prostaglandin levels, contribute to this assumption.

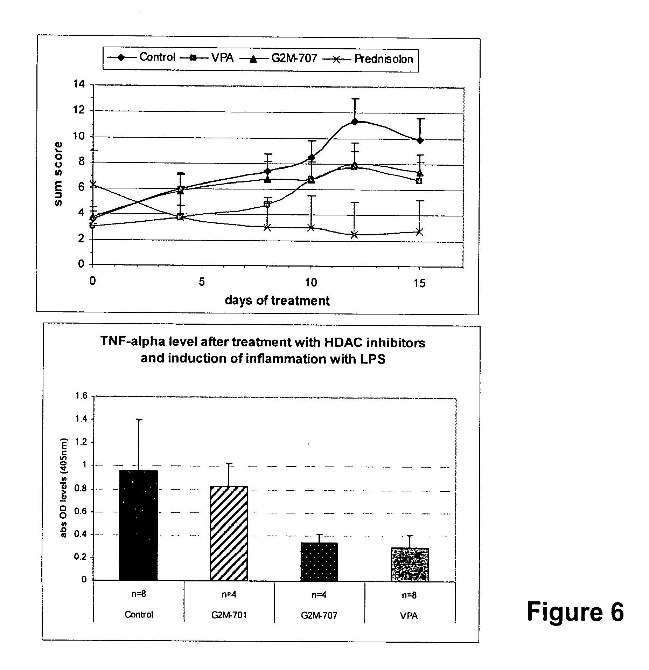

To evaluate the ability of HDAC inhibitors to inhibit soluble TNF-α secretion in vivo, an acute LPS-induced inflammation model in 3H1 mice was used. The mice were injected i.p. with substances tested 1h before the i.p. injection of LPS. Blood samples were drawn 1 h after LPS stimulus and the TNF-α levels in the serum were determined using a TNF-α ELISA assay. As shown in figure 6 (lower panel), VPA and G2M-707 pretreatment led to a 60% reduction in absolute TNF-α serum levels in comparison to the control mice.

This experiment clearly shows, that HDAC inhibitors are potent inhibitors of TNF-α levels in vivo and may be used to treat inflammatory diseases which respond to a reduction of levels in Cox-2 and TNF-α.

In detail, mice were treated with VPA (400 mg/kg/d, n=8), G2M-701 (1 mg/mouse/d, n=4), G2M-707 (1 mg/mouse/d, n=4) or with 200µl PBS (control, n=8) 1 h before inflammation was induced with 50 µg LPS (Sigma) per mouse. One hour after the LPS treatment, blood was taken by cardiac puncture and serum was isolated. The serum was tested in a TNF-α sandwich ELISA module from Bender MedSystems. The assay was performed as described in the manufacturer's manual. ABTS was used as a substrate and measuring was accomplished with a 96-well plate reader at a wavelength of 405 nm. Absolute OD levels at 405 nm are given.

To evaluate this anti-inflammatory potency of HDAC inhibitors in a therapeutic in vivo inflammation model we applied VPA and G2M-707 to mice that had developed collagen induced arthritis (CIA).

Figure 6 (upper panel) shows the result of treatment with VPA or G2M-707 in such a model for Rheumatoid Arthritis (RA). Both drugs efficiently reduced clinical severity scores (sum score) as compared to the control group, and the efficacy was maintained through the course of the treatment. Prednisolon, a corticosteroid drug in clinical use for RA, was used as a positive control in this study.
Each data point represents the average of 9 (VPA group), 8 (G2M-707 group) or 4 (Prednisolon group) animals, respectively. P < 0,05 (two-sample t-test; Control vs. VPA-treated and Control vs. Prednisolon-treated animals).

In detail, in this therapeutic CIA model, DBA/1 female mice of age 7 weeks were immunized with 100 µg of chicken type II collagen (Chondrex) in CFA. Development of arthritis started 21 to 28 days after immunization and reached an incidence of 93% after 6 weeks. The severity was medium to high and reached a mean score of 10.3 (maximum score 15) in untreated animals. The mice were monitored daily for signs of arthritis using an established scoring system. At the first sign of arthritis, the affected mice were assigned to a treatment group. The mice were treated for 15 days with vehicle control or VPA at 2 x 400 mg/kg/d i.p. or G2M-707 at 2 x 1 mg/mouse/d i.p. or Prednisolon at 2 x 20 mg/kg/d (i.p.). Clinical severity of arthritis was assessed based on the appearance of each paw and subjectively graded on a scale of 0 to 4. The scores of each limb were summed, giving a maximum severity score of 16. The scoring system was as follows: 0, no arthritis; 1, redness of paw and one or two swollen digits; 2, mild to moderate swelling of the entire paw; 3, extensive swelling of the entire paw; 4, extreme swelling of entire paw and beginning of ankylosis. After onset of disease the animals were scored 3 times a week.

## Claims

1. The use of a histone deacetylase inhibitor in combination with a NSAID for the manufacture of a medicament for the treatment or prevention of a disease **characterized in that** the histone deacetylase HDAC-2 is upregulated in tissue affected by said disease.

2. The use according to claim 1, wherein at least part of the tissue affected by said disease (a) harbors at least one mutation in the APC gene, or (b) harbors at least one mutation in the β-catenin gene which leads to a gain of function of β-catenin or a stabilization or enhanced half life of the β-catenin protein, or (c) shows upregulation or enhanced function of c-myc, and/or (d) shows mutations or alterations of the Wnt pathway that lead to HDAC-2 upregulation.

3. The use of claim 1 or 2 wherein the disease is an inherited condition leading to cancer, cancer predisposing disorders, or an inflammatory disorder.

4. The use of any one of claims 1 to 3 wherein the inherited condition is Familial Adenomatous Polyposis.

5. The use of any one of claims 1 to 4 wherein the NSAID is a Cyclooxygenase inhibitor.

6. The use of any one of claims 1 to 5 wherein the NSAID is a Cyclooxygenase-2 inhibitor.

7. The use of any one of claims 1 to 6 wherein the NSAID is selected from salicylates, arylalkanoic acids, 2-arylpropionic acids, N-arylanthranilic acids, oxicams such as meloxicam and piroxicam, coxibs such as celecoxib, valdecoxib, lumiracoxib, etoricoxib, and rofecoxib, sulphonanilides, indomethacin, sulindac, aspirin, flurbiprofen, ibuprofen, naproxen drugs, and derivatives thereof.

8. The use according to any one of claims 1 to 7 wherein the histone deacetylase inhibitor is a compound of formula I 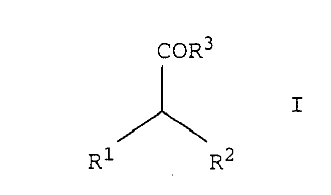 wherein R¹ and R² independently are a linear or branched, saturated or unsaturated, aliphatic C₃₋₂₅ hydrocarbon chain which optionally comprises one or several heteroatoms and which may be substituted, R³ is hydroxyl, halogen, alkoxy or an optionally alkylated amino group, or a pharmaceutically acceptable salt thereof.

9. The use according to claim 8, wherein R¹ and R² independently are a linear or branched C₃₋₂₅ hydrocarbon chain which optionally comprises one double or triple bond.

10. The use according to any one of claims 1 to 9, wherein the histone deacetylase inhibitor is valproic acid or a pharmaceutically acceptable salt thereof.

11. A use according to any one of claims 1 to 7 wherein the histone deacetylase inhibitor is selected from the group consisting of hydroxamic acid derivatives, benzamides, pyroxamides and derivatives thereof, microbial metabolites exhibiting HDAC inhibitory activity, fatty acids and derivatives thereof, cyclic tetrapeptides, peptidic compounds, HDAC class III inhibitors and SIRT inhibitors or a pharmaceutical acceptable salt thereof.

12. The use according to any one of claims 1 to 7 wherein the inhibitor of histone deacetylases is selected from the group consisting of hydroxamic acid derivatives (such as NVP-LAQ824, LBH-589, Trichostatin A (TSA), Suberoyl anilide hydroxamic acid, CBHA, G2M-701, G2M-702, G2M-707, Pyroxamide, Scriptaid, Cl-994, CG-1521, Chlamydocin, Biaryl hydroxamate, A-161906, Bicyclic aryl-N-hydroxycarboxamides, PXD-101, Sulfonamide hydroxamic acid,) TPX-HA analogue (CHAP), Oxamflatin, Trapoxin, Depudecin, Apidicin, benzamides, (such as MS-27-275, and MGCD0103) butyric acid and derivatives thereof, Pivanex (Pivaloyloxymethyl butyrate), trapoxin A, Depsipeptide (FK-228) and related peptidic compounds, Tacedinaline and MG2856 or a pharmaceutical acceptable salt thereof.

13. The use of claim 1 to 12 wherein the disease is estrogen receptor-dependent breast cancer, estrogen receptor-independent breast cancer, hormone receptor-dependent prostate cancer, hormone receptor-independent prostate cancer, brain cancer, renal cancer, colon cancer, colorectal cancer, pancreatic cancer, bladder cancer, esophageal cancer, stomach cancer, genitourinary cancer, gastrointestinal cancer, uterine cancer, ovarian cancer, astrocytomas, gliomas, skin cancer, squamous cell carcinoma, Keratoakantoma, Bowen disease, cutaneous T-Cell Lymphoma, melanoma, basal cell carcinoma, actinic keratosis; ichtiosis; acne, acne vulgaris, sarcomas, Kaposi's sarcoma, osteosarcoma, head and neck cancer, small cell lung carcinoma, non-small cell lung carcinoma, leukemias, lymphomas and/or other blood cell cancers.

14. The use of claim 1 to 12 wherein the disease is thyroid resistance syndrome, diabetes, thalassemia, cirrhosis, protozoal infection, rheumatoid arthritis, rheumatoid spondylitis, all forms of rheumatism, osteoarthritis, gouty arthritis, multiple sclerosis, insulin dependent diabetes mellitus, non-insulin dependent diabetes, asthma, rhinitis, uveithis, lupus erythematoidis, ulcerative colitis, Morbus Crohn, inflammatory bowel disease, chronic diarrhea, psoriasis, atopic dermatitis, bone disease, fibroproliferative disorders, atherosclerosis, aplastic anemia, DiGeorge syndrome, Graves' disease, epilepsia, status epilepticus, Alzheimer's disease, depression, schizophrenia, schizoaffective disorder, mania, stroke, mood-incongruent psychotic symptoms, bipolar disorder, affective disorders, meningitis, muscular dystrophy, multiple sclerosis, agitation, cardiac hypertrophy, heart failure, reperfusion injury and/or obesity.

15. The use according to any one of claims 1 to 14 wherein the medicament is applied by intravenous, intramuscular, subcutaneous, topical, oral, nasal, intraperitoneal or suppository based administration.

16. The use according to any of claims 1 to 15 wherein the different drug compounds are administered separately in the form of two individual drugs or in an administrative form which contains both drugs in a single application unit.

17. A pharmaceutical composition comprising valproic acid or a pharmaceutically acceptable salt thereof, celecoxib and at least one pharmaceutically acceptable excipient or diluent.

18. A pharmaceutical kit comprising as a first component valproic acid or a pharmaceutically acceptable salt thereof, and as a second component celecoxib.

19. A pharmaceutical composition comprising valproic acid or a pharmaceutically acceptable salt thereof, Sulindac and at least one pharmaceutically acceptable excipient or diluent.

20. A pharmaceutical kit comprising as a first component valproic acid or a pharmaceutically acceptable salt thereof, and as a second component Sulindac.

21. A pharmaceutical composition comprising valproic acid or a pharmaceutically acceptable salt thereof, aspirin and at least one pharmaceutically acceptable excipient or diluent.

22. A pharmaceutical kit comprising as a first component valproic acid or a pharmaceutically acceptable salt thereof, and as a second component aspirin.

23. A pharmaceutical composition comprising valproic acid or a pharmaceutically acceptable salt thereof, lbuprofen and at least one pharmaceutically acceptable excipient or diluent.

24. A pharmaceutical kit comprising as a first component valproic acid or a pharmaceutically acceptable salt thereof, and as a second component lbuprofen.

25. A pharmaceutical composition comprising valproic acid or a pharmaceutically acceptable salt thereof, fenamic acid and at least one pharmaceutically acceptable excipient or diluent.

26. A pharmaceutical kit comprising as a first component valproic acid or a pharmaceutically acceptable salt thereof, and as a second component fenamic acid.

27. A pharmaceutical composition comprising valproic acid or a pharmaceutically acceptable salt thereof, piroxicam and at least one pharmaceutically acceptable excipient or diluent.

28. A pharmaceutical kit comprising as a first component valproic acid or a pharmaceutically acceptable salt thereof, and as a second component piroxicam.

29. A pharmaceutical composition comprising PXD101 or a pharmaceutically acceptable salt thereof, Celecoxib and at least one pharmaceutically acceptable excipient or diluent.

30. A pharmaceutical kit comprising as a first component PXD101 or a pharmaceutically acceptable salt thereof, and as a second component Celecoxib.
